# EUROPEAN PATENT APPLICATION

(11) **EP 4 084 163 A1**
(43) Date of publication of application: **02.11.2022**
(21) Application number: 21171268.2
(22) Date of filing: 29.04.2021
(51) Int. Cl.: H01M 8/04014, C25B 1/04, C25B 3/03, H01M 8/04089, H01M 8/04119, H01M 8/0432, H01M 8/0438, H01M 8/04791, H01M 8/0612, H01M 8/0637, H01M 8/0662, H01M 8/18, H01M 8/124

(54) **FUEL CELL SYSTEM AND METHOD FOR OPERATING THE SAME**

(71) Applicant: Technische Universität München, 80333 München (DE)
(72) Inventor: HERRMANN, Stephan, 81925 München (DE); FISCHER, Felix, 80336 München (DE); SPLIETHOFF, Hartmut, 82140 Olching (DE); HAUCK, Maximilian, 81369 München (DE); WEINRICH, Jeremias, 94315 Straubing (DE)
(74) Representative: Hoefer & Partner Patentanwälte mbB

(57) **Abstract**

The present invention concerns a fuel cell system (1) arranged for the conversion of pure hydrogen comprising a) at least one fuel cell (2) comprising an anode (2a), a cathode (2b) and an electrolyte (2c) provided between the anode (2a) and cathode (2b), the fuel cell (2) further being arranged for an internal reformation of methane, b) an anode exhaust conduit (4) connecting an anode outlet (2d) and a methanation unit (3), the methanation unit (3) being capable of producing methane from anode exhaust, and c) a methanation unit exhaust conduit (11) connecting a methanation unit exit and a fuel conduit (6), wherein the fuel introduced into a fuel conduit inlet (7) of the fuel conduit (6) is pure hydrogen, and wherein the amount of methane produced in the methanation unit (3) is equal to the amount of methane reformed inside of the fuel cell (2) so that the content of methane cycling through the fuel cell system (1) is constant.

## Description

The present invention relates to a fuel cell system having an increased total efficiency and a method for operating the fuel cell system with increased total efficiency.

Fuel cell systems, especially high temperature fuel cell systems (e.g. SOFC) with an operation temperature of around 600-800 °C, are widely used in the prior art for generating electric power produced by the redox reaction of a fuel stream and an oxidant stream. Especially preferred is hydrogen as the fuel gas since it does not include carbon-containing substances, which can lead to carbon deposition inside the fuel cells.

However, fuel cell systems known in the prior art typically have a maximum fuel utilization of about 70 to 80 % only, these fuel cell systems showing a low total efficiency and generate up to 60 % waste heat. Due to the heat produced by the electrochemical reaction inside the fuel cell, cooling needs to be carried out. This is typically done by using the air supplied to the cathode for this purpose, which consumes a lot of energy for the air blower.

It is also known in the prior art, that operating a high temperature fuel cell on a mixture of hydrocarbon-based fuels (e.g. natural gas) and water steam leads to a reduced amount of waste heat due to endothermic reforming of the hydrocarbon fuel with the water steam and therefore often higher efficiency system efficiency compared to hydrogen based systems. In the prior art the addition of water steam or other oxygen containing substances, such as carbon dioxide, in large quantities is necessary when operating on hydrocarbon fuels to prevent carbon deposition related damage of the fuel cell. However, since the water steam (and/or CO₂) is a reaction product of the fuel cell reaction, it is disadvantageous to add large amounts of water steam to the fuel, since this reduces the Nernst potential and thereby the efficiency and/or power density of the fuel cell significantly.

It is therefore an object underlying the present invention to provide a highly efficient fuel cell system for power generation. Moreover, it is an object of the present invention to provide a method of operating a fuel cell system with increased fuel utilization and an increased total efficiency. It is further an object of the present invention to provide a method for operating a reversible fuel cell system in the fuel cell mode with increased fuel utilization and an increased total efficiency, which is also capable of operating in an electrolysis mode, thereby producing pure hydrogen from water steam and electricity (any fuel cell system from hereon can equivalently be considered a reversible fuel cell system of this kind, specifically while being operated in the fuel cell mode).

The object is achieved by the independent claims. The sub-claims contain advantageous embodiments of the present invention.

Accordingly, the inventive fuel cell system is arranged for the conversion of pure hydrogen fed to the (reversible) fuel cell system and comprises at least one fuel cell, which, according to a preferred embodiment, is a solid oxide fuel cell (SOFC). The fuel cell itself comprises an anode, a cathode and an electrolyte, which is provided between the anode and cathode. The fuel cell is further arranged for an internal reformation of methane. "Internal" in this sense means "within the fuel cell". In other words, a reformation section forms part of the fuel cell and is thus, provided within the fuel cell (inside of the fuel cell), and is particularly located near and/or at the electrochemical reaction zone of the fuel cell and therefore in direct thermal contact with the fuel cell reaction zone.

Fuel is supplied to an anode inlet of the anode via a fuel conduit and the fuel introduced into the fuel conduit via a fuel conduit inlet is pure hydrogen (which, of course, depending on its origin may contain unavoidable impurities up to 5 Vol.-%). An anode exhaust conduit is provided, which connects an anode outlet of the fuel cell and a methanation unit. The methanation unit is capable of producing methane from anode exhaust. More particular, the anode exhaust contains hydrogen and carbon containing substances, and therefore, the methanation unit can produce CH₄ out of a mixture containing CO, CO₂ and hydrogen. The methanation unit further comprises a methanation unit exit, and a methanation unit exhaust conduit connects the methanation unit exit and the fuel conduit. In other words, the methanation unit exhaust conduit is coupled to the fuel conduit so that methane produced in the methanation unit is supplied to the fuel conduit and mixed with the pure hydrogen supplied to the fuel conduit via the fuel conduit inlet.

When operating said fuel cell system, fuel containing hydrogen and methane (= mixture) is supplied to the anode and methane contained in the fuel is reformed inside of the fuel cell. Since the reformation reaction is endothermal, heat produced by the fuel cell reaction (consumption of hydrogen at the anode side) is absorbed and used in the reformation reaction. In other words, heat produced in the fuel cell by the fuel cell reaction can be effectively consumed by carrying out the reformation reaction inside of the fuel cell. This makes cooling of the fuel cell by use of air supplied to the cathode less necessary, i.e. less electric power for driving a fan or the like is used to supply air to the cathode as the air is no longer mainly used for cooling the fuel cell.

However, in order to have such efficient internal cooling, which, due to saving of electric power for operating a fan or the like at the cathode side, improves the total efficiency of the fuel cell system, it is necessary to supply to the fuel cell a respective amount of methane. According to the present invention, this methane, once fed to the fuel cell system, is not supplied from the outside so that methane is consumed by operating the fuel cell system, but instead the methane used for the reformation is generated in the methanation unit, which is one of the essential components of the fuel cell system.

Moreover, the amount of methane produced in the methanation unit is controlled such that it is equal to the amount of methane reformed internally inside the fuel cell so that the content of methane cycling through the fuel cell system is constant. This results in a closed methane cycle. When the amount of reformed methane is equal to the amount of methane produced in the methanation unit, the respective amount of reformed methane can be adjusted so that no or only a minimum of cooling energy has to be supplied to the fuel cell via e.g. the cathode.

The cycling of a constant amount of methane through the fuel cell system by using a fuel cell arranged for internal reformation and a methanation unit controlled as outlined above, provided in the anode exhaust conduit, achieves high energy output and thus, a high total degree of efficiency of the fuel cell system.

Although not explicitly mentioned above, the fuel cell of the inventive fuel cell system further comprises an oxidant conduit for supplying oxidant, like oxygen or air, to the cathode via a cathode inlet. Further provided is an oxidant exhaust conduit.

The above-described fuel cell system further preferably comprises means to control the ratio of hydrogen to methane in the fuel mixture depending to the fuel cell operating temperature and pressure, such that carbon deposition is thermodynamically prevented without the necessity of presence of water steam in the fuel mixture, i.e. without adding water to the fuel supplied to the fuel cell, wherein the means are capable of adjusting the ratio according to the following values and intermediate values by linear interpolation between the values given:
- atmospheric pressure, 550°C, volume ratio H₂:CH₄ > 1.7; or
- 2 bar pressure absolute, 550°C, volume ratio H₂:CH₄ > 1; or
- 5 bar pressure absolute, 550°C, volume ratio H₂:CH₄ > 0.63; or
- atmospheric pressure, 600°C, volume ratio H₂:CH₄ > 2.5; or
- 2 bar pressure absolute, 600°C, volume ratio H₂:CH₄ > 2; or
- 5 bar pressure absolute, 600°C, volume ratio H₂:CH₄ > 1.5; or
- atmospheric pressure, 650°C, volume ratio H₂:CH₄ > 5.5 ; or
- 2 bar pressure absolute, 650°C, volume ratio H₂:CH₄ > 3; or
- 5 bar pressure absolute, 650°C, volume ratio H₂:CH₄ > 1.5; or
- atmospheric pressure, 700°C, volume ratio H₂:CH₄ > 10; or
- 2 bar pressure absolute, 700°C, volume ratio H₂:CH₄ > 5; or
- 5 bar pressure absolute, 700°C, volume ratio H₂:CH₄ > 2.4.

This is advantageous with respect to the above-mentioned negative effect of water steam presence in fuel fed to the fuel cell anode on the Nernst potential of the fuel cell and therefore leads to a higher efficiency of the fuel cell. The methane content of the fuel supplied to the anode is then reformed step by step with only the water steam formed during the electrochemical oxidation of the hydrogen in the fuel instead of excess water steam present in the fuel feed in the prior art. This has the further advantage, that the reformation reaction is spread out continuously along the fuel cell reaction zone instead of leading to a large temperature drop directly at the anode inlet.

According to a further preferred embodiment, the methanation unit exhaust conduit comprises first heat transferring means (like e.g. a heat exchanger) for transferring heat from methanation unit exhaust to the fuel (mixture) supplied to the anode inlet. Since the methanation reaction is an exothermic reaction, transferring heat contained in the methanation product to the fuel in the fuel conduit increases the heat utilization rate and thus, contributes to the overall increase of the total degree of efficiency.

In order to further increase the electric power production by the fuel cell system, the fuel cell system advantageously further comprises a steam circuit, capable of producing electric power using water vapor. The steam circuit is thermally coupled to the methanation unit, in so far as the methanation unit reaction zone, where the exothermic methanation occurs, is directly or indirectly arranged for transferring heat to the steam circuit, preferably to the evaporation section of the steam circuit. Using this transferred heat, water vapor can be produced easily, which then can be used to drive a turbine or the like, which then produces electric power.

In order to increase the efficiency of the production of electric power via the steam circuit, the steam circuit preferably comprises at least one turbine.

Further preferable in view of an increase of the thermal utilization level, the anode exhaust conduit between the anode outlet and the methanation unit of the fuel cell system may further comprise second heat transferring means (like e.g. a heat exchanger) for transferring heat from the anode exhaust to the fuel or fuel mixture to be supplied to the anode via the fuel conduit.

Since the fuel cell reaction is exothermic, heat included in the anode exhaust is consequently used directly to heat up fuel or fuel mixture, which greatly supports the endothermal reformation reaction and contributes to facilitation of the fuel cell reaction.

According to a preferred embodiment, the fuel cell system further comprises a water removal and/or water condenser unit coupled to the methanation unit exhaust conduit, which may be provided downstream to the first heat transferring means, if present. The water removal and/or water condenser unit is capable of reducing the steam content of the methanation product so that the fuel mixture fed to the fuel cell only contains a small amount of residual steam. High amounts of steam in the fuel mixture greatly reduce the electrochemical efficiency of the fuel cell reaction by reducing the Nernst voltage potential.

Therefore, removing water cycling through the fuel cell system contributes to an increase in the electrochemical efficiency and thus, also contributes to an increase in the total efficiency degree of the fuel cell system.

When the methane supplied to the anode inlet corresponds to the methane produced in the methanation unit, besides the initial methane content during operation no additional methane is supplied to the fuel cell system and, according to this embodiment, the carbon related emissions can be kept at a minimum, comprising only of minor gasket leakages.

The total efficiency degree of the inventive fuel cell system can be further improved according to an embodiment, in which the content of methane cycling through the fuel cell system is set such that at least 30%, preferably at least 50% and more preferably at least 70% of the heat of the fuel cell reaction is consumed by the reformation reaction.

In view of an increase in the flexibility and functionality of the fuel cell system, it is preferable that the fuel cell is a reversible fuel cell and can be operated in a fuel cell mode and additionally an electrolysis mode.

In the electrolysis mode, the fuel cell is preferably capable of converting a mixture of hydrogen and water steam into a mixture richer in hydrogen without the presence of carbon containing gases. This effectively enables the production of pure hydrogen in the system, which, after condensation of residual water steam, can be provided to external hydrogen users or stored to be re-utilized in the fuel cell operating mode.

A further aspect of the present invention relates to a method of operating the above-described fuel cell system. Accordingly, in view of the components and functionality of the fuel cell system, additional reference is made to the annotations and explanations provided above.

The method comprises feeding pure hydrogen into the fuel conduit inlet of the fuel conduit. The hydrogen serves as the only fuel to be converted in the overall balance of the fuel cell system so that there are overall no carbon dioxide emissions from the system. In the fuel conduit, the pure hydrogen is mixed with methanation unit exhaust fed from the methanation unit exhaust conduit, preferably after water has been removed from the methanation unit exhaust. Said methanation unit exhaust comprises hydrogen and methane as the main components. Then, the obtained mixture is fed to the anode inlet. In the fuel cell, methane contained in the mixture is then reformed with water steam primarily originating from the electrochemical oxidation of hydrogen inside of the fuel cell so that hydrogen and carbon oxides are produced in an endothermic reformation reaction. A fuel cell reaction is carried out in the fuel cell, wherein hydrogen serves as the primary fuel, which is converted. Anode exhaust exiting the anode outlet is then supplied to the methanation unit via the anode exhaust conduit and methane is generated out of the anode exhaust in the methanation unit. The amount of methane produced in the methanation unit is equal to the amount of methane reformed in the internal reforming so that the content of methane cycling through the fuel cell system is constant and a closed methane cycle is provided.

Since the methane used in the above operating method is kept at a constant amount and no new methane is fed via the fuel conduit, carbon dioxide emissions of the fuel cell system can be prevented efficiently. Furthermore, since methane is reformed within the fuel cell, heat produced by the fuel cell reaction can be used for carrying out the endothermal reformation reaction so that the fuel cell is directly cooled by the internal reformation reaction. Therefore, cooling mainly by distributing high amounts of air to the cathode, which consumes a lot of electric power for e.g. a fan or the like, can be avoided. Accordingly, heat generated in the fuel cell system is effectively used and not wasted, which increases the total efficiency degree of the fuel cell system.

According to a further preferred embodiment, the operating conditions of the fuel cell are controlled by adjusting the ratio of cycling hydrogen and methane depending on operating pressure and temperature inside the fuel cells as follows, such that carbon deposition is thermodynamically not favored even when no water vapor is present, whereby linear interpolation between the given values provides exemplarily an approximate borderline for carbon deposition, which must not be undercut:
- atmospheric pressure, 550°C, volume ratio H₂:CH₄ > 1.7; or
- 2 bar pressure absolute, 550°C, volume ratio H₂:CH₄ > 1; or
- 5 bar pressure absolute, 550°C, volume ratio H₂:CH₄ > 0.63; or
- atmospheric pressure, 600°C, volume ratio H₂:CH₄ > 2.5; or
- 2 bar pressure absolute, 600°C, volume ratio H₂:CH₄ > 2; or
- 5 bar pressure absolute, 600°C, volume ratio H₂:CH₄ > 1.5; or
- atmospheric pressure, 650°C, volume ratio H₂:CH₄ > 5.5 ; or
- 2 bar pressure absolute, 650°C, volume ratio H₂:CH₄ > 3; or
- 5 bar pressure absolute, 650°C, volume ratio H₂:CH₄ > 1.5; or
- atmospheric pressure, 700°C, volume ratio H₂:CH₄ > 10; or
- 2 bar pressure absolute, 700°C, volume ratio H₂:CH₄ > 5; or
- 5 bar pressure absolute, 700°C, volume ratio H₂:CH₄ > 2.4.

According to a preferable embodiment and in order to further increase the heat efficiency and heat utilization, the method comprises transferring heat from methanation unit exhaust to the mixture to be fed to the anode inlet. This improves the thermal efficiency of the fuel cell system.

Alternatively or additively, the method may comprise a step of transferring heat from the methanation unit to a steam circuit to support the production of electric power using water vapor in the steam circuit. According to this step, the electric power production can be increased directly. E.g. a turbine may be driven by water vapor produced by heating water with heat generated during the methanation reaction.

Further preferable, the method includes a step of removing water and/or condensing water in the methanation unit exhaust.

The removing of water or the condensing of water is preferably carried out after transferring heat from methanation unit exhaust to the mixture to be fed to the anode inlet, wherein in particular, the removing of water or the condensing of water is controlled such that a content of water vapor in the mixture at the anode inlet is less than 10 Vol%, preferably less than 3 Vol%, relative to the total volume flow of the mixture supplied to the anode inlet. This guarantees a high efficiency and power generation.

Further preferable the content of methane cycling through the fuel cell system is set such that at least 30%, preferably at least 50% and more preferably at least 70% of the heat of the fuel cell reaction is consumed by the reformation reaction. The higher the consumption of heat produced in the fuel cell reaction, the less thermal energy is wasted in the form of warm air exhaust, which contributes to the total efficiency degree of the fuel cell system.

The method may further comprise a step of cycling an additional amount of hydrogen through the fuel cell system, wherein the cycling additional amount of hydrogen is not consumed.

More preferably, the fuel cell system may comprise more than one fuel cell and the sum of the current flowing through all individual fuel cells (current density times the total cell area in the plurality of fuel cells in the system) is set such that it is equal to the number of available electrons in the hydrogen fed into the fuel cell system through the fuel conduit inlet (2 moles of electrons per mole of hydrogen fed per unit of time), subtracted by potential hydrogen losses through leakages. This resembles a fuel utilization of 100 % of the hydrogen fed through the fuel conduit subtracted by potential hydrogen leakages.

According to a further preferable embodiment, the fuel cell may be a reversible fuel cell, wherein when the fuel cell is operated in an electrolysis mode hydrogen instead of carbon-containing species is produced and hydrogen and optionally water vapour are cycled through the fuel cell. Thereby pure hydrogen can be produced in the same reversible fuel cell system.

According to an alternative embodiment, in the electrolysis mode, methane, or a mixture of methane and hydrogen, may be produced and water and carbon dioxide are added to the system in a volume ratio of around H₂O : CO₂ = 4 : 1. Thereby methane can be produced in a highly effective manner, which can be used for other purposes or stored.

Further preferable, in the electrolysis mode the methanation unit provides water vapour for the electrolysis reaction when methane is produced.

Further details, advantages and characteristics of the present invention will be explained with respect to the following description of the embodiments in light of the enclosed Figure. The Figure shows:

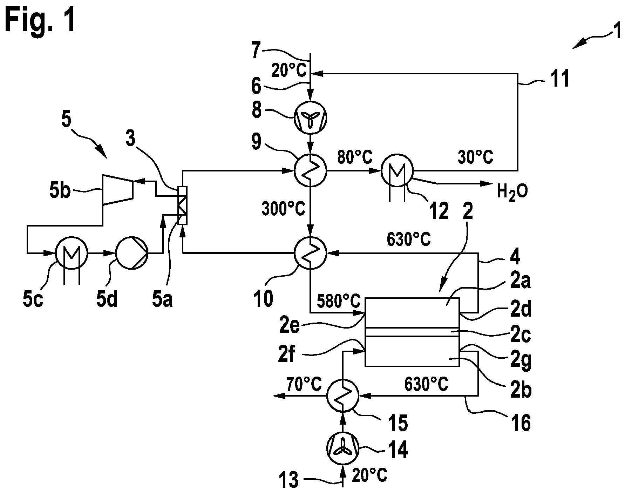

- Fig. 1: a schematic diagram of a fuel cell system according to an embodiment.

The present invention is described with reference to the following Figure. Herein, all essential elements and components of the inventive fuel cell are shown. All other elements and components have been omitted to increase the understanding of the present invention. Any temperature values given are only provided as illustration for better understanding and do not represent any restriction to the values shown.

In detail, Fig. 1 shows a fuel cell system 1, which is arranged for the conversion of pure hydrogen. The fuel cell system comprises one fuel cell 2 comprising an anode 2a, a cathode 2b and an electrolyte 2c provided between the anode 2a and cathode 2b. The fuel cell 2 is further arranged for internal reformation in or around the anode (2a). "Internal reformation" means that the conversion of methane, and thus, the reformation reaction of methane, is carried out within the fuel cell 2, i.e. inside the fuel cell and thus, in direct thermal contact with the reaction zone of the fuel cell 2.

The fuel cell system 1 further comprises a methanation unit 3. The methanation unit 3 is configured to produce methane from anode exhaust. Accordingly, an anode outlet 2d and the methanation unit 3 are connected via anode exhaust conduit 4.

The methanation unit 3 is thermally coupled to a steam cycle 5 and therefore, a third heat exchanger 5a forms part of the methanation unit 3. The steam cycle 5 comprises a turbine 5b for generating electric power, which is driven by water vapor. Further provided in the steam cycle 5 are a water condenser or water separator 5c and a pump 5d.

A fuel conduit 6 connects a fuel conduit inlet 7 with anode inlet 2e. In the fuel conduit 6 a blower 8 is provided and downstream thereof, a first heat exchanger 9 and a second heat exchanger 10 are provided.

A methanation unit exhaust conduit 11 connects the methanation unit 3 with the fuel conduit 6 upstream the blower 8. In the methanation unit exhaust conduit 11 a water condenser or water separator 12 is provided to separate water from the methanation unit exhaust.

When operating the fuel cell system 1, pure hydrogen is fed to the fuel conduit 6 via fuel conduit inlet 7. "Pure hydrogen" means hydrogen with a purity of at least 95 Vol.-% and preferably at least 99.5 Vol.-%, the remainder being unavoidable impurities. In the fuel conduit 6, the hydrogen is mixed with methanation unit exhaust, which mainly contains methane and hydrogen. As an example, 4 mol of hydrogen per second might be fed through the fuel conduit inlet 7, while the methanation unit exhaust may feed 1 mol hydrogen per second relative to 1.25 mol methane per second to the fuel conduit 6. The mixture of 5 mol/s hydrogen and 1.25 mol/s methane is then compressed in blower 8 and reaches the first heat exchanger 9. Heat exchanger 9 transfers heat from the methanation unit exhaust to the mixture of fuel including mainly hydrogen and methane, so that the mixture is pre-heated for a first time and the temperature is increased from about environmental temperature (20 °C) to e.g. 300 °C.

The fuel mixture then enters the second heat exchanger 10. Here, heat is transferred from anode exhaust to the mixture so that at the anode inlet 2e, the temperature of the mixture is e.g. about 580 °C. Such high temperature is typically necessary for the appropriate operation of Solid Oxide Fuel Cells.

Anode exhaust leaves the anode outlet 2d at a high temperature of about e.g. 630 °C, this heat being used to further pre-heat the mixture to be supplied to the anode inlet 2e in the second heat exchanger 10. The then pre-cooled anode exhaust enters the methanation unit 3 and methane is generated by the exothermic methanation reaction. Since the anode exhaust is entering the methanation unit 3 at lower temperature, the methanation reaction, which is exothermic, is further promoted.

The methanation reaction produces methane and heat and the heat is transferred to the steam cycle 5. The heat can be used to produce water vapor from water and the water vapor can drive a turbine so that extra electric power can be produced and the reaction heat is more effectively used.

Sensible heat of the methanation exhaust can be used to pre-heat the fuel mixture containing hydrogen and methane in the fuel conduit 6 by using the first heat exchanger 9. The temperature of the methanation unit exhaust may then drop to e.g. 80 °C and subsequently water is condensed and separated from the methanation unit exhaust in water condenser or water separator 12. Due to the dependence of saturation pressure and temperature, this leads to a further decrease of the temperature of the methanation unit exhaust to a value ideally close to environmental temperature in order to achieve a low water steam content. The water-reduced methanation exhaust is then again mixed with the external hydrogen feed entering the fuel conduit 6 via fuel conduit inlet 7.

For the sake of completeness, it is outlined that the cathode 2b comprises a cathode inlet 2f and a cathode outlet 2g. Oxidant gas, like e.g. pure oxygen or air is supplied to the cathode inlet 2g via oxidant conduit 13. In the oxidant conduit 13 a blower 14 is arranged as well as a heat exchanger 15. The heat exchanger uses heat from the cathode exhaust supplied via a cathode exhaust conduit 16 to pre-heat the oxidant in the oxidant conduit 13, so that thermal energy at the cathode side of the fuel cell 2 is effectively used.

The fuel cell system 1 is highly efficient. Depending on the operating conditions, such as temperature, pressure, single pass fuel utilization ratio, etc., the fuel cell electrical efficiency is about 70 %, which means that 70% of the heating value of the hydrogen fuel fed through inlet 7 is obtained as electric energy in the fuel cell 2. Additionally, around 10 % electric power can be produced by the steam cycle 5. However, the excellent thermal balance is only achieved since an amount of methane produced in the methanation unit is equal to the amount of methane reformed in the fuel cell so that the content of methane cycling through the fuel cell system is constant. The so-called closed methane cycle is controlled such that the heat produced in the fuel cell 2 is highly consumed by the reformation reaction inside the fuel cell. Accordingly, thermal and consequently electric energy for extra cooling of the fuel cell 2 by e.g. supplying high amounts of air to the cathode, can be saved. Furthermore, the generation of methane from the anode exhaust again produces heat, which can be used to produce the additional electric current in the steam cycle, so that the total efficiency of the fuel cell system 1 is exceptionally high.

While embodiments of the invention have been illustrated and described, it is not intended that these embodiments illustrate and describe all possible form of the invention. The words used in the specification are words of description rather than limitation, and it is understood that various changes may be made without departing from the spirit and the scope of the invention.

### Reference signs

- 1: fuel cell system
- 2: fuel cell
- 2a: anode
- 2b: cathode
- 2c: electrolyte
- 2d: anode outlet
- 2e: anode inlet
- 2f: cathode inlet
- 2g: cathode outlet
- 3: methanation unit
- 4: anode exhaust conduit
- 5: steam cycle
- 5a: third heat exchanger
- 5b: turbine
- 5c: water condenser or water separator
- 5d: pump
- 6: fuel conduit
- 7: fuel conduit inlet
- 8: blower
- 9: first heat exchanger
- 10: second heat exchanger
- 11: methanation unit exhaust conduit
- 12: water condenser or water separator
- 13: oxidant conduit
- 14: blower
- 15: heat exchanger
- 16: cathode exhaust conduit

## Claims

1. A fuel cell system (1) arranged for the conversion of pure hydrogen comprising:
- at least one fuel cell (2) comprising an anode (2a), a cathode (2b) and an electrolyte (2c) provided between the anode (2a) and cathode (2b), the fuel cell (2) being arranged for an internal reformation of methane,
- an anode exhaust conduit (4) connecting an anode outlet (2d) and a methanation unit (3), the methanation unit (3) being capable of producing methane from anode exhaust,
- a methanation unit exhaust conduit (11) connecting a methanation unit exit and a fuel conduit (6),
wherein the fuel introduced into a fuel conduit inlet (7) of the fuel conduit (6) is pure hydrogen, and
wherein the amount of methane produced in the methanation unit (3) is equal to the amount of methane reformed inside of the fuel cell (2) so that the content of methane cycling through the fuel cell system (1) is constant.

2. The fuel cell system (1) of claim 1, further comprising means to control the ratio of hydrogen to methane in the fuel mixture depending to the fuel cell operating temperature and pressure, such that carbon deposition is thermodynamically prevented without the necessity of presence of water steam in the fuel mixture, wherein the means are capable of adjusting the ratio according to the following values and intermediate values by linear interpolation between the values given:
• atmospheric pressure, 550°C, volume ratio H₂:CH₄ > 1.7; or
• 2 bar pressure absolute, 550°C, volume ratio H₂:CH₄ > 1; or
• 5 bar pressure absolute, 550°C, volume ratio H₂:CH₄ > 0.63; or
• atmospheric pressure, 600°C, volume ratio H₂:CH₄ > 2.5; or
• 2 bar pressure absolute, 600°C, volume ratio H₂:CH₄ > 2; or
• 5 bar pressure absolute, 600°C, volume ratio H₂:CH₄ > 1.5; or
• atmospheric pressure, 650°C, volume ratio H₂:CH₄ > 5.5 ; or
• 2 bar pressure absolute, 650°C, volume ratio H₂:CH₄ > 3; or
• 5 bar pressure absolute, 650°C, volume ratio H₂:CH₄ > 1.5; or
• atmospheric pressure, 700°C, volume ratio H₂:CH₄ > 10; or
• 2 bar pressure absolute, 700°C, volume ratio H₂:CH₄ > 5; or
• 5 bar pressure absolute, 700°C, volume ratio H₂:CH₄ > 2.4.

3. The fuel cell system (1) of claim 1 or 2, the methanation unit exhaust conduit (11) comprising first heat transferring means (9) for transferring heat from methanation unit exhaust to the fuel supplied to the anode inlet (2e) and/or
the fuel cell system further comprising a steam circuit (5), capable of producing electric power using water vapor, the methanation unit exhaust conduit (11) comprising third heat transferring means (5a) for transferring heat from methanation unit exhaust to the steam circuit (5), and/or the fuel cell system further comprising a water removal and/or water condenser unit (12) coupled to the methanation unit exhaust conduit (11) and/or
wherein the methane supplied to the anode inlet (2e) corresponds to the methane produced in the methanation unit (3) and/or
wherein the content of methane cycling through the fuel cell system (1) is set such that at least 30%, preferably at least 50% and more preferably at least 70% of the heat of the fuel cell reaction is consumed by the reformation reaction.

4. The fuel cell system (1) according to any one of the preceding claims, wherein the fuel cell (2) is a reversible fuel cell and can be operated in a fuel cell mode according to the preceding claims and additionally an electrolysis mode.

5. The fuel cell system (1) according to claim 4, wherein in the electrolysis mode the fuel cell (2) is capable of converting a mixture of hydrogen and water steam into a mixture richer in hydrogen without the presence of carbon containing gases.

6. A method of operating the fuel cell system (1) according to any one of the preceding claims, the method comprising:
a) feeding pure hydrogen into the fuel conduit inlet (7) of the fuel conduit (6);
b) mixing the pure hydrogen with methanation unit exhaust in the methanation unit exhaust conduit (11) and feeding the obtained mixture to the anode inlet (2e);
c) reforming methane contained in the mixture inside of the fuel cell (2);
d) carrying out a fuel cell reaction in the fuel cell (2); and
e) generating methane out of the anode exhaust in the methanation unit (3);
wherein the amount of methane produced in the methanation unit (3) is equal to the amount of methane reformed inside of the fuel cell (2) so that the content of methane cycling through the fuel cell system (1) is constant.

7. The method of claim 6, wherein the operating conditions of the fuel cell are controlled by adjusting the ratio of cycling hydrogen and methane depending on operating pressure and temperature inside the fuel cells as follows, such that carbon deposition is thermodynamically not favored even when no water vapor is present, whereby linear interpolation between the given values provides exemplarily an approximate borderline for carbon deposition, which must not be undercut:
• atmospheric pressure, 550°C, volume ratio H₂:CH₄ > 1.7; or
• 2 bar pressure absolute, 550°C, volume ratio H₂:CH₄ > 1; or
• 5 bar pressure absolute, 550°C, volume ratio H₂:CH₄ > 0.63; or
• atmospheric pressure, 600°C, volume ratio H₂:CH₄ > 2.5; or
• 2 bar pressure absolute, 600°C, volume ratio H₂:CH₄ > 2; or
• 5 bar pressure absolute, 600°C, volume ratio H₂:CH₄ > 1.5; or
• atmospheric pressure, 650°C, volume ratio H₂:CH₄ > 5.5 ; or
• 2 bar pressure absolute, 650°C, volume ratio H₂:CH₄ > 3; or
• 5 bar pressure absolute, 650°C, volume ratio H₂:CH₄ > 1.5; or
• atmospheric pressure, 700°C, volume ratio H₂:CH₄ > 10; or
• 2 bar pressure absolute, 700°C, volume ratio H₂:CH₄ > 5; or
• 5 bar pressure absolute, 700°C, volume ratio H₂:CH₄ > 2.4.

8. The method of claim 6 or 7, comprising transferring heat from methanation unit exhaust to the mixture to be fed to the anode inlet (2e).

9. The method of any one of claims 6 to 8, comprising transferring heat from the methanation unit to a steam circuit (5) to support the production of electric power using water vapor in the steam circuit (5).

10. The method of any one of claims 6 to 9, comprising removing water and/or condensing water in the methanation unit exhaust, wherein the removing of water or the condensing of water is preferably carried out after transferring heat from methanation unit exhaust to the mixture to be fed to the anode inlet (2e), wherein in particular, the removing of water or the condensing of water is controlled such that a content of water vapor in the mixture at the anode inlet (2e) is less than 10 Vol%, preferably less than 3 Vol%, relative to the total volume flow of the mixture supplied to the anode inlet (2e).

11. The method of any one of claims 6 to 10, comprising setting the content of methane cycling through the fuel cell system (1) such that at least 30%, preferably at least 50% and more preferably at least 70% of the heat of the fuel cell reaction is consumed by the reformation reaction.

12. The method of any one of claims 6 to 11, further comprising cycling an additional amount of hydrogen through the fuel cell system, wherein the cycling additional amount of hydrogen is not consumed.

13. The method of any one of claims 6 to 12, wherein the sum of the current flowing through all individual fuel cells is set such that it is equal to the number of available electrons in the hydrogen fed into the system through the fuel conduit inlet per unit of time, subtracted by potential hydrogen losses through leakages.

14. The method of any one of claims 6 to 13, wherein when the fuel cell (2) is operated in an electrolysis mode hydrogen is produced and hydrogen and optionally water vapour are cycled through the fuel cell (2) or
wherein when the fuel cell (2) is operated in an electrolysis mode methane is produced and water and carbon dioxide are added to the system in a volume ratio of H₂O : CO₂ = 4 : 1.

15. The method of any one of claims 6 to 14, wherein when the fuel cell (2) is operated in an electrolysis mode the methanation unit provides water vapour for the electrolysis reaction.
